# EUROPEAN PATENT APPLICATION

(11) **EP 1 647 251 A1**
(43) Date of publication of application: **19.04.2006**
(21) Application number: 05256311.1
(22) Date of filing: 11.10.2005
(51) Int. Cl.: A61G 12/00

(54) **Medical appliance support with accessory tracks**

(30) Priority: 14.10.2004 US 618931 P
(71) Applicant: Hill-Rom Services, Inc., Wilmington, DE 19801 (US)
(72) Inventor: Hamberg, Stephen R., Cincinnati, Ohio 45251 (US); Reinke, Christian H., York, South Carolina 29745 (US)
(74) Representative: Findlay, Alice Rosemary

(57) **Abstract**

A service head (40) comprises a housing (100) having an internal passageway (150) and a member-receiving cavity (112) which opens through an outer surface (102) thereof, and an extruded member (122) received in the member-receiving cavity (112). The extruded member (122) has an accessory support track (132) that is accessible through an opening (142) in the outer surface (102). The accessory support track (132) is configured to support at least one patient care accessory (80). The passageway (150) is configured to allow routing of at least one service line (152) therethrough to couple to the at least one patient care accessory (80).

## Description

The present disclosure relates to a service head for supporting patient care equipment, such as patient monitors, infusion pumps, service connectors, etc., in a hospital room adjacent a patient support, such as a hospital bed, stretcher, and the like.

Hospitalized patients often require patient care equipment to be in close proximity during their hospital stay. Such patient care equipment may include any one or more of the following: service connectors, infusion pumps, heart monitors, defibrillators, equipment monitors, and the like, many of which directly connect to the patient via lines or tubes. Some of the service connectors may be electrical outlets to supply electrical power. Some of the service connectors may be medical gas outlets to provide medical gases, such as oxygen, nitrogen, and air. Some of the service connectors may be negative pressure outlets to supply vacuum. Some of the service connectors may be data communication ports to receive and transmit data, such as, for example, audio, video, and patient information.

In accordance with one aspect of the present invention, a service head may comprise a housing having at least one member-receiving cavity which opens through an outer surface thereof, and at least one extruded member received in the at least one member-receiving cavity. The at least one extruded member may have an accessory support track that is accessible through an opening in the outer surface. The accessory support track may be configured to support at least one patient care accessory. The housing may have an internal passageway for routing at least one service line therethrough for connection to the at least one patient care accessory.

An outer surface of the at least one extruded member may be substantially coplanar with the outer surface of the housing having the opening. The housing may be a casting, such as, for example, a machined aluminum sand casting. The at least one extruded member may be an aluminum extrusion.

The housing may have a first generally vertical portion having the at least one member-receiving cavity, and a second portion extending outwardly from an upper end of the first generally vertical portion. The second portion may be coupled to a radial arm for pivoting movement therewith.

The at least one extruded member may extend substantially the entire length of the housing. A bottom surface of the housing may be substantially coplanar with a bottom surface of the at least one extruded member. The housing may have a plurality of service connectors. The accessory track may have a T-shaped cross section. The patient care equipment may be coupled to the accessory track by mounting brackets. The mounting brackets may have T-shaped portions that slidingly fit within the T-shaped accessory track. The service connectors may be any one or more of the following: electrical outlets, emergency power outlets, low voltage outlets, medical gas outlets, data ports, and the like.

The at least one member-receiving cavity may comprise a first member-receiving cavity opening through a first outer surface of the housing and a second member-receiving cavity opening through a second outer surface of the housing. The at least one extruded member may comprise a first extruded member received in the first member-receiving cavity and a second extruded member received in the second member-receiving cavity. The first extruded member may have a first accessory support track that is accessible through a first opening in the first outer surface. The second extruded member may have a second accessory support track that is accessible through a second opening in the second outer surface. The accessory support tracks may be configured to support one or more patient care accessories.

The housing may have a front wall, a back wall, and side walls. The first and second member-receiving cavities may open through the front and back walls of the housing, respectively. The housing may have at least one cross member extending between the side walls of the housing through the internal passageway. The first and second extruded members may be fastened to the at least one cross member on the opposite sides thereof.

In accordance with another aspect, a service head may comprise a single-piece extruded member having a plurality of faces and a plurality of corners. The plurality of faces and the plurality of corners may form a continuous outer surface circumscribing an internal passageway. At least one corner of the extruded member may have an accessory support track that is accessible through an opening in the at least one corner. The accessory track may be configured to support at least one patient care accessory. The passageway may be configured to allow routing of at least one service line therethrough to couple to the at least one patient care accessory.

The extruded member may have four faces and four corners. At least one face and at least one corner of the extruded member may define planes which are generally at a 45° angle. The at least one face and the at least one corner may each have an accessory support track. The extruded member and the internal passageway may each have a generally square cross section. The extruded member may have at least one longitudinally extending opening adjacent to one of the corners of the extruded member for securing a coupler, such as a 2-axes pivot coupler, to the extruded member. The at least one opening may comprise four openings adjacent to the four corners of the extruded member. The openings may be threaded.

The extruded member may be coupled to a radial arm for pivoting movement therewith. The at least one patient care accessory may be a monitor coupled to the accessory track. A support bracket may be coupled to the accessory track for supporting the monitor. A releasable lock may be movable between a first position locking the support bracket to the accessory track and a second position unlocking the support bracket from the accessory track. The service head may comprise a counterbalanced arm, coupled to the support bracket, for supporting the monitor. A low voltage cable may extend through the internal passageway for connection to the monitor. A handle may be coupled to the accessory track for manipulating the service head.

The invention will now be further described by way of example with reference to the accompanying drawings in which:
Fig. 1 is a perspective view of a patient care equipment support system showing a service head supported by a pair of radial arms, and carrying a monitor, a pair of infusion pumps and a handle,
Fig. 2 is a perspective view of the Fig. 1 system showing a plurality of electrical outlets coupled to an upwardly inclining underside of a cantilevered portion of the service head, and the attachment of the service head to a 2-axes pivot coupler supported by the radial arms,
Fig. 3 is a cut-away perspective view of the Fig. 1 service head showing a housing having first and second member-receiving cavities on the opposite sides thereof, first and second extruded members received in the first and second member-receiving cavities and having respective accessory support tracks, and an internal passageway extending through the housing through which a plurality of service lines are passed for connection to patient care accessories, such as the monitor and the infusion pumps, supported by the accessory tracks,
Fig. 4 is a perspective view of the Fig. 1 service head showing an opening in a mounting flange of the service head through which the service lines enter the internal passageway in the service head,
Figs. 5 and 6 are front and rear perspective views of the Fig. 1 service head,
Fig. 7 is a perspective view of the Fig. 1 service head showing a handle coupled to the first accessory track,
Figs. 8 is a top view of the Fig. 1 service head showing the service head supporting a monitor,
Fig. 9 is a cross sectional plan view of the Fig. 1 service head showing the housing and the first and second extruded members attached to the housing on the opposite sides thereof,
Fig. 10 is a perspective view of a second embodiment of a patient care equipment support system showing a service head comprising an extruded member supported by a pair of radial arms, and carrying a monitor and a handle,
Figs. 11 and 12 are side and rear perspective views of the Fig. 10 service head,
Fig. 13 is a perspective view of the Fig. 10 extruded member,
Fig. 14 is a cross sectional plan view of the Fig. 10 extruded member showing the accessory tracks on the faces and corners,
Fig. 15 is a perspective view of a Fairfield rail assembly showing a Fairfield rail and two mounting brackets for attaching the Fairfield rail to an accessory track,
Figs. 16-18 are respectively side, top and rear views of the Fig. 15 Fairfield rail assembly,
Fig. 19 is a rear perspective view of the Fig. 15 Fairfield rail assembly,
Fig. 20 is a perspective view of a second embodiment of Fairfield rail assembly,
Figs. 21-23 are respectively side, top and rear views of the Fig. 20 Fairfield rail assembly, and
Fig. 24 is a rear perspective view of the Fig. 20 Fairfield rail assembly.

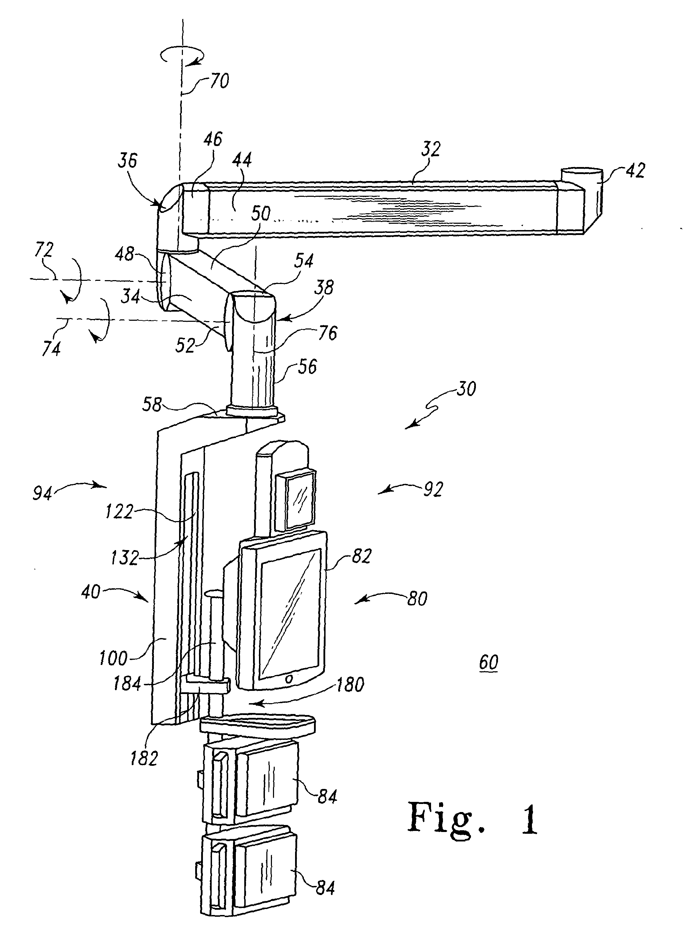

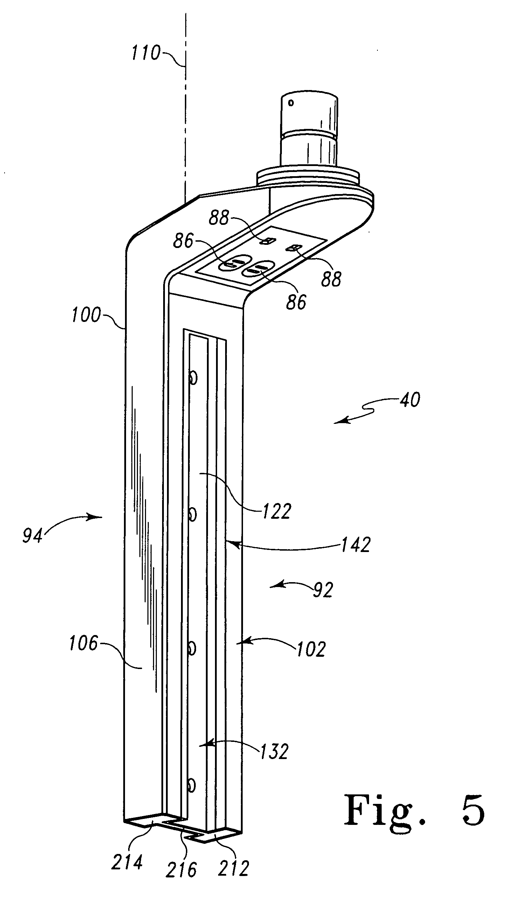

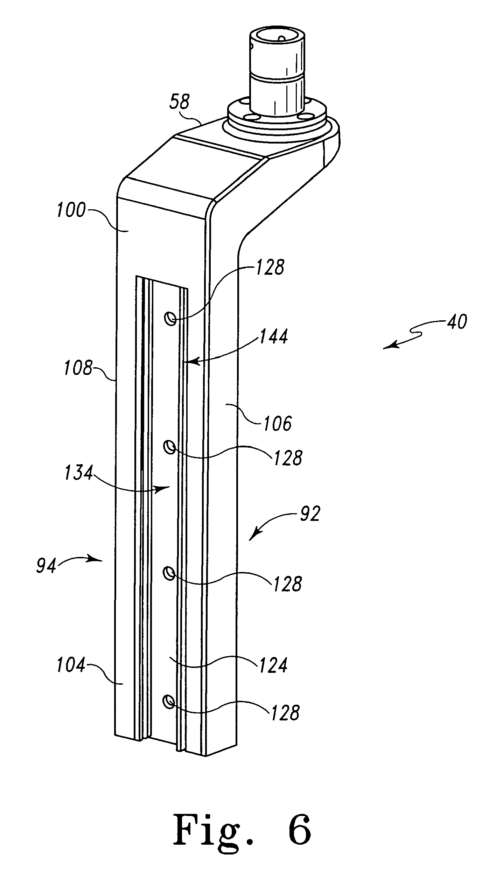

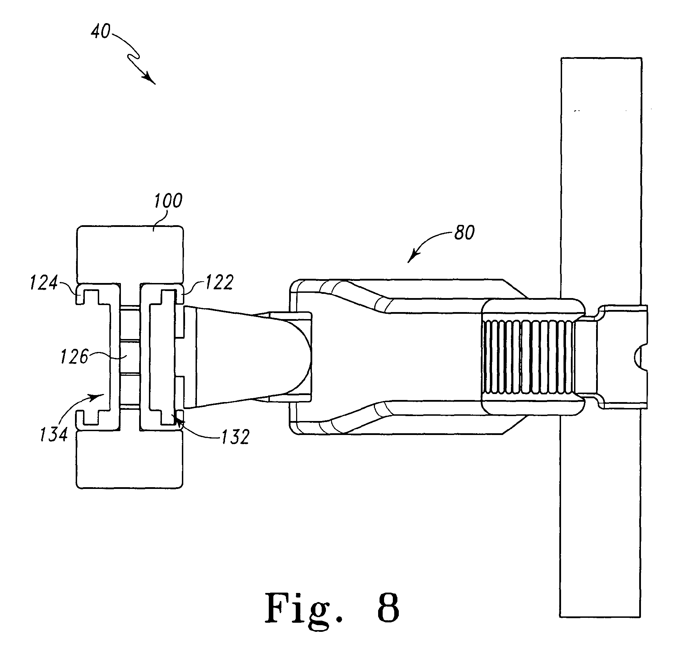

Referring to Figs. 1-9, an illustrative patient care equipment support system 30 comprises first and second radial arms 32, 34, first and second 2-axes pivot couplers 36, 38, and a service head 40. A proximal end 42 of the radial arm 32 is coupled to a ceiling or wall mounted support structure (not shown). A distal end 44 of the radial arm 32 is coupled to a proximal end 46 of the pivot coupler 36. A distal end 48 of the pivot coupler 36 is coupled to a proximal end 50 of the radial arm 34. A distal end 52 of the radial arm 34 is coupled to a proximal end 54 of the pivot coupler 38. A distal end 56 of the pivot coupler 38 is coupled to a proximal end 58 of the service head 40. The pivot coupler 36 allows rotation of the radial arm 34 about a vertical axis 70 and about a horizontal axis 72 relative to the radial arm 32. The pivot coupler 38 allows rotation of the service head 40 about a horizontal axis 74 and about a vertical axis 76 relative to the radial arm 34. The radial arms 32, 34 and the pivot couplers 36, 38 allow the service head 40 to be positioned at any desirable location and in any desirable orientation within their respective ranges of movement.

The support structure for the radial arms 32, 34 may be coupled to a ceiling or a wall of a patient room 60 in a hospital or a healthcare facility. Alternatively, the support structure may extend upwardly from a floor of the patient room 60. Although the system 30 is disclosed in the context of being useful for a patient room 60, the system 30 may very well be used in other settings such as, for example, intensive care rooms, operating rooms, physicians offices, nursing homes, and the like.

The service head 40 is configured to support patient care equipment 80 (also referred to herein as patient care accessories). In Fig. 1, the patient care equipment 80 comprises a patient monitor 82, infusion pumps 84, a pair of line voltage outlets 86, and a pair of low voltage outlets 88. However, the patient care equipment 80 may very well include any one or more of the following: defibrillators, equipment monitors, medical gas outlets to provide medical gases (such as oxygen, nitrogen, and air), negative pressure outlets to supply vacuum, data communication ports to transmit and receive data (such as, for example, audio, video, and patient information), emergency power outlets, and the like. The electrical outlets 86 supply electrical power at 110 volts. The low voltage power outlets 88 supply electrical power at 12/24 volts.

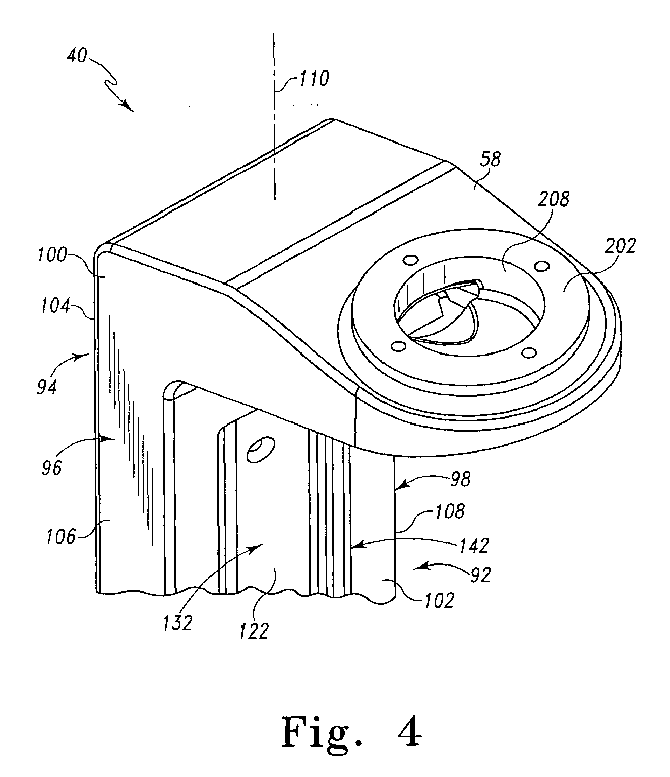

As shown, for example, in Fig. 4, the service head 40 has a front side 92, a back side 94, a left side 96, and a right side 98. As used in this description, the phrase "front side 92" will be used to denote the side of any referred-to object that is positioned to lie nearest the front side 92 of the service head 40, the phrase "back side 94" will be used to denote the side of any referred-to object that is positioned to lie nearest the back side 94 of the service head 40, the phrase "left side 96" will be used to denote the side of any referred-to object that is positioned to lie nearest the left side 96 of the service head 40, and the phrase "right side 98" will be used to denote the side of any referred-to object that is positioned to lie nearest the right side 98 of the service head 40.

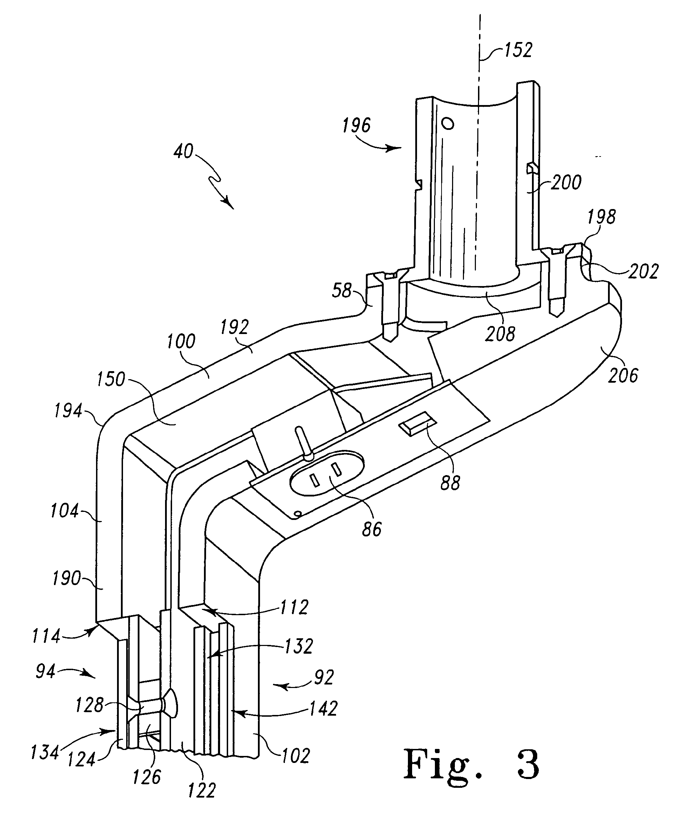

As shown in Fig. 4, the service head 40 comprises a housing 100 having a front wall 102, a back wall 104, a pair of side walls 106, 108, and a longitudinal axis 110. As shown in Fig. 3, the housing 100 includes a first member-receiving cavity 112 opening through the front wall 102 of the housing 100, and a second member-receiving cavity 114 opening through the back wall 104 of the housing 100. A first extruded member 122 is received in the first member-receiving cavity 112, and a second extruded member 124 received in the second member-receiving cavity 114. As shown, for example, in Fig. 3, a plurality of cross members 126 extend between the side walls 106, 108 of the housing 100. The first and second extruded members 122, 124 are attached to the cross members 126 on the opposite sides 92, 94 thereof by suitable fasteners, such as screws 128. The outer surfaces of the front and back extruded members 122, 124 are substantially coplanar with the outer surfaces of the front and back walls 102, 104 of the housing 100, respectively.

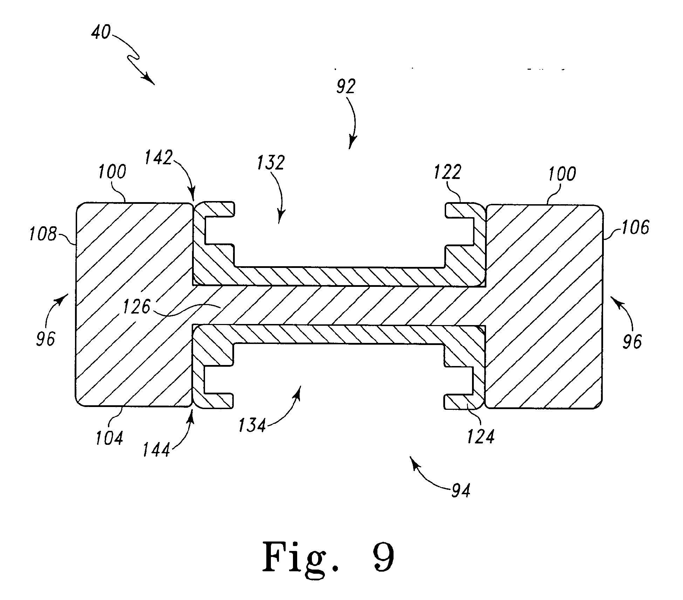

As shown in Fig. 9, the first extruded member 122 has a first accessory support track 132 that is accessible through a first opening 142 in the front wall 102. The second extruded member 124 has a second accessory support track 134 that is accessible through a second opening 144 in the back wall 104. The accessory tracks 132, 134 are configured to support patient care equipment 80, such as the monitor 82 and the infusion pumps 84. The housing 100 has an internal passageway 150 through which a plurality of service lines 152, shown diagrammatically in Fig. 3, are routed for connection to the patient care equipment 80 supported on tracks 132, 134. The extruded members 122, 124 have a plurality of apertures (not shown) that extend between the respective tracks 132, 134 and the internal passageway 150 in the housing 100. The service lines 152 pass through the apertures in the extruded members 122, 124 for connection to the associated patient care equipment 80.

Fig. 9 shows GCX-type accessory tracks 132, 134 configured to be used with GCX mounting brackets marketed by GCX Corporation, 3875 Cypress Drive, Petaluma, CA 94954-5635. The patient care equipment 80 is attached to the tracks 132, 134 via GCX mounting brackets. Typically, each mounting bracket has a complementary head portion (not shown) that slidably fits within the tracks 132, 134. The mounting brackets may each include a releasable lock (not shown) that is movable between a first position to lock the mounting bracket to the associated track 132, 134 and a second position to unlock the mounting bracket from the associated track 132, 134. Although GCX-type accessory tracks 132, 134 are used in the embodiment of Figs. 1-9, tracks having other profiles may very well be used instead.

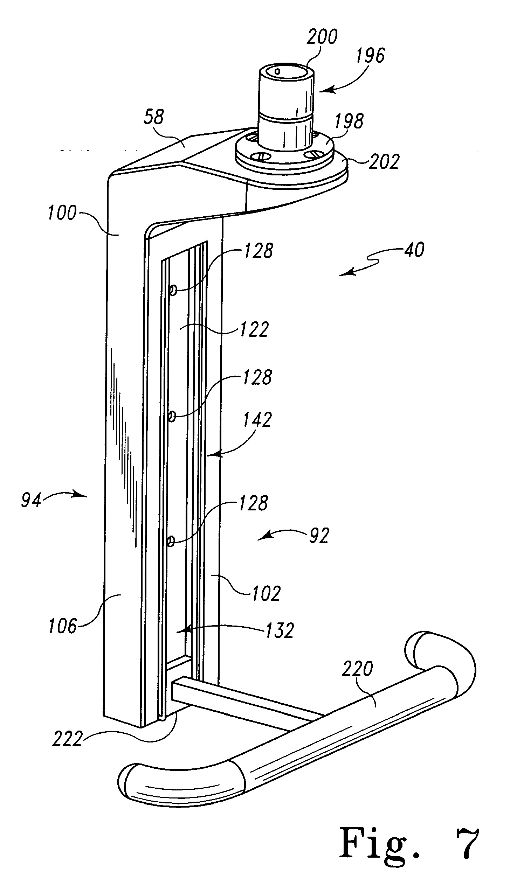

Referring to Fig. 1, the monitor 82, the infusion pumps 84, and the handle 90 are all coupled to the accessory track 132 via a GCX-type mounting bracket 180. The mounting bracket 180 comprises a horizontal support arm 182 having a proximal end coupled to the track 132 and a distal end coupled to a vertical mounting post 184. The monitor 82, the infusion pumps 84, and the handle 90 are coupled to the mounting post 184 by suitable fittings. The vertical position of the mounting bracket 180 along the track 132 can be adjusted by releasing the associated lock coupling the mounting bracket 180 to the track 132, moving the mounting bracket 180 along the track 132 to a desired position, and then relocking the lock. Illustratively, the service lines 152 extend through the first radial arm 32, the first pivot coupler 36, the second radial arm 34, the second pivot coupler 38, the passageway 150 in the housing 100 for connection to the patient care equipment 80, such as the monitor 82 and the infusion pumps 84. The service lines 152 include a low voltage cable coupled to the monitor 82. Appropriate shielding may be provided around the high voltage cable to reduce interference between the low voltage cable and the high voltage cable. Fig. 7 shows a second embodiment of the handle 90, designated by numeral 220, coupled to the track 132 via a mounting bracket 222.

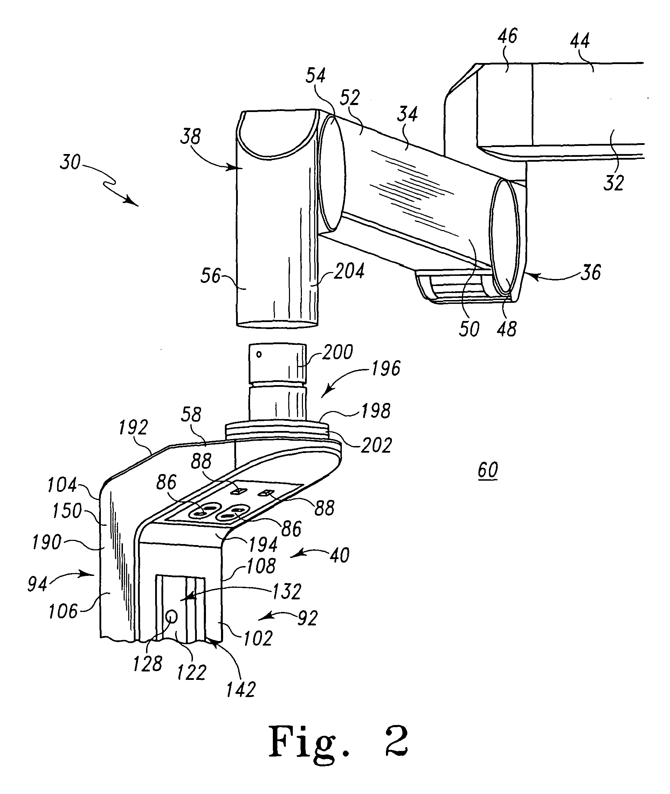

As shown in Fig. 2, the housing 100 has a first generally vertical portion 190 and a second generally horizontal portion 192 extending outwardly from an upper end 194 of the first generally vertical portion 190. The horizontal portion 192 is coupled to the pivot coupler 38 by means of a fitting 196. The fitting 196 includes a first flange portion 198 and a second tube portion 200 that extends generally upwardly from the flange portion 198. The flange portion 198 is attached to a mounting flange 202 on the topside of the horizontal portion 192 by suitable fasteners, such as set screws. The tube portion 200 is received in a socket portion 204 of the pivot coupler 38 for rotation about the vertical axis 76.

As shown in Fig. 3, the service lines 152 enter the passageway 150 in the housing 100 through a central bore 208 in the mounting flange 202. The service outlets 86, 88 are coupled to the underside 206 of the second generally horizontal portion 192, which angles upwardly toward the front side 92 of the service head 40. The extruded members 122, 124 extend substantially the entire length of the housing 100, and the bottom surfaces 212, 214 of the extruded members 122, 124 are substantially coplanar with the bottom surface 216 of the housing 100. Illustratively, the housing 100 is a casting, such as a machined aluminum sand casting, and the extruded members 122, 124 are aluminum extrusions.

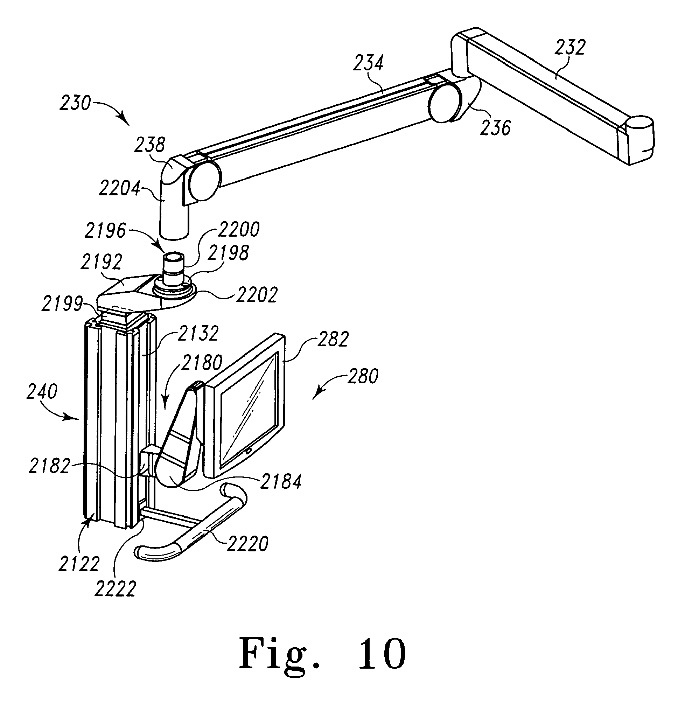

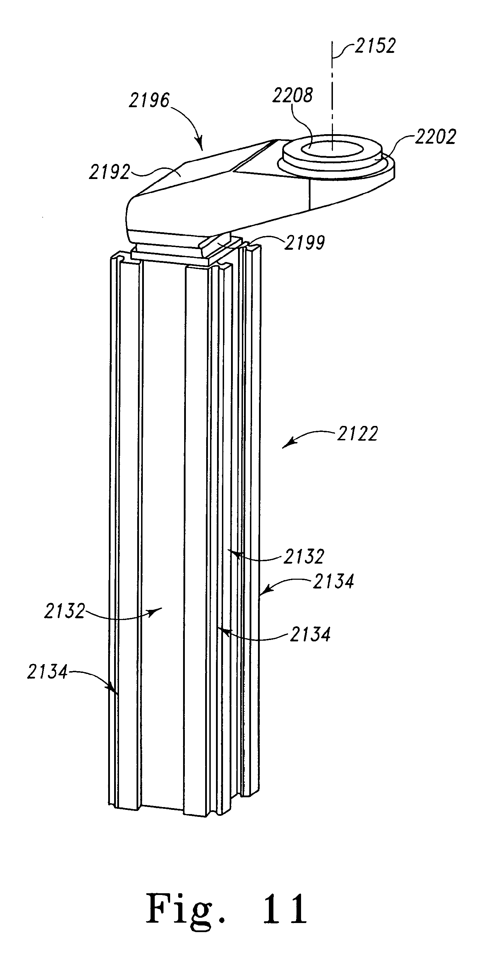

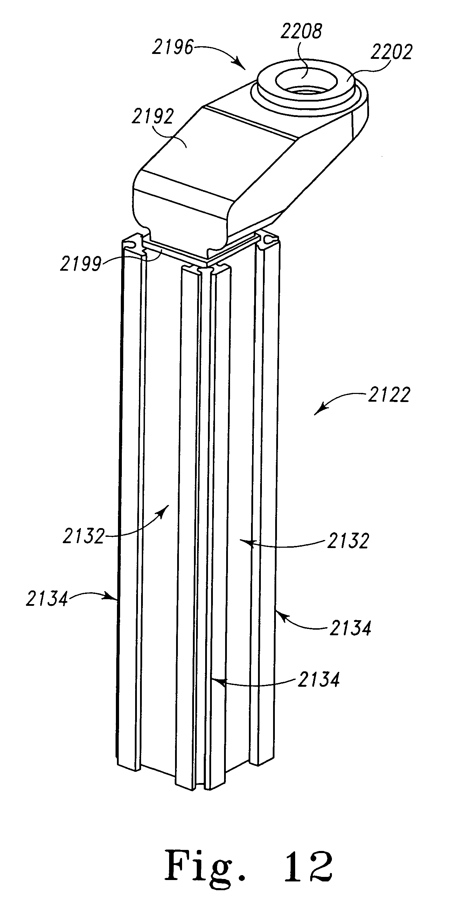

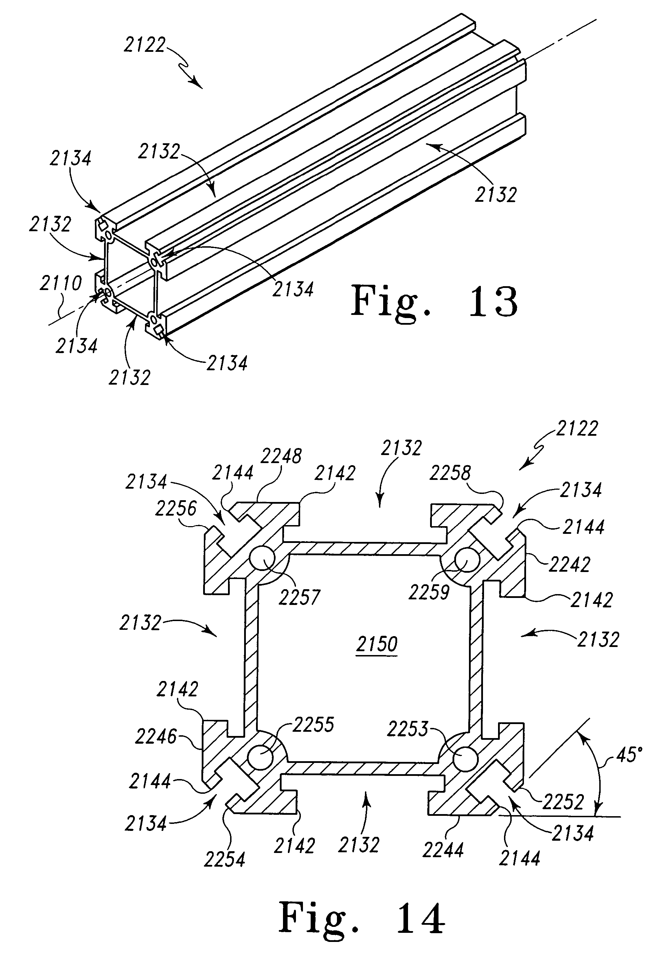

Figs. 10-14 illustrate a second embodiment of the service head 40. Like elements of the two embodiments have generally similar reference numbers. Thus, in the second embodiment, numeral 230 designates the patient care equipment support system, numerals 232, 234 designate the radial arms, numerals 236, 238 designate the 2-axes pivot couplers, and numeral 240 designates the service head. As shown in Figs. 13 and 14, the service head 240 comprises a single-piece extruded member 2122 having a plurality of faces 2242, 2244, 2246, 2248 and a plurality of corners 2252, 2254, 2256, 2258. The plurality of faces 2242, 2244, 2246, 2248 and the plurality of corners 2252, 2254, 2256, 2258 form a continuous outer surface circumscribing an internal passageway 2150. Each face 2242, 2244, 2246, 2248 has an accessory support track 2132 that is accessible through an opening 2142 in the associated face 2242, 2244, 2246, 2248. Each corner 2252, 2254, 2256, 2258 has an accessory support track 2134 that is accessible through an opening 2144 in the associated corner 2252, 2254, 2256, 2258. The tracks 2132, 2134 are configured to support patient care equipment 280, such as a monitor 282. The faces 2242, 2244, 2246, 2248 and the corners 2252, 2254, 2256, 2258 of the extruded member 2122 have a plurality of apertures (not shown) that extend between the respective tracks 2132, 2134 and the internal passageway 2150 in the extruded member 2122. The service lines 2152, shown diagrammatically in Fig. 11, pass through the internal passageway 2150 and through the respective apertures in the extruded member 2122 for connection to the associated patient care equipment 280.

The patient care equipment 280 is attached to the tracks 2132, 2134 via suitable mounting brackets. Typically, each mounting bracket has a complementary head portion (not shown) that slidably fits within the tracks 2132, 2134. The mounting brackets may each include a releasable clamp (not shown) that is movable between a first position to lock the mounting bracket to the associated track 2132, 2134 and a second position to unlock the mounting bracket from the associated track 2132,2134.

As shown in Fig. 14, the illustrative extruded member 2122 has four faces or sides 2242, 2244, 2246, 2248 and four corners 2252, 2254, 2256, 2258. The faces 2242, 2244, 2246, 2248 and the corners 2252, 2254, 2256, 2258 have generally planar or flat surfaces. The extruded member 2122 and the passageway 2150 each has a generally square cross section. Each face 2242, 2244, 2246, 2248 defines a plane which is generally at a 45° angle relative to a plane defined by each adjacent corner 2252, 2254, 2256, 2258. Thus, the face 2242 and the adjacent corners 2252, 2258 define planes which are generally at a 45° angle, and the face 2244 and the adjacent corners 2254, 2252 define planes which are generally at a 45° angle.

As shown in Fig. 14, each accessory track 2132, 2134 has a generally T-shaped cross section as viewed from the top side. The T-shaped cross section comprises a first generally rectangular base portion having a first width and a second generally rectangular head portion having a second width smaller than the first width. In one illustrative embodiment, the dimensions of the side tracks 2132 are: the width of the base portion is 2.530 inches (about 1.0 centimeter), the depth of the base portion is 0.270 inch (about 0.11 centimeter), the width of the head portion is 2.060 inches (about .81 centimeter), and the depth of the head portion is 0.326 inch (about 0.13 centimeter). All dimensions are plus/minus 0.01 inch (about 0.0059 centimeter). In another illustrative embodiment, the tracks 2132, like the tracks 132, are configured for supporting GCX-type instrument mounting brackets.

The corner tracks 2134 are smaller than the side tracks 2132. In one illustrative embodiment, the corner tracks 2134 are configured to be used with mounting brackets marketed by Hill-Rom Company, Inc., Batesville, Indiana. The corner tracks 2134 are also referred to as Hill-Rom profile or AP (Architectural Products) tracks. Illustratively, the dimensions of the corner tracks 2134 are: the width of the base portion is 0.674 inch (about 0.27 centimeter), the depth of the base portion is 0.326 inch (about 0.13 centimeter), the width of the head portion is 0.326 inch (about 0.13 centimeter), and the depth of the head portion is 0.230 inch (about 0.09 centimeter). Although Hill-Rom profile tracks are used for corner tracks 2134, tracks having other profiles may very well be used.

Referring to Fig. 10, the monitor 282 is coupled to the accessory track 2132 via a mounting bracket 2180. The mounting bracket 2180 comprises a horizontal support arm 2182 having a proximal end coupled to the accessory track 2132 and a distal end coupled to a proximal end of a counterbalanced arm 2184. A distal end of the counterbalanced arm 2186 is coupled to the monitor 282. The mounting bracket 2180 includes a releasable lock (not shown). The vertical position of the mounting bracket 2180 along the track 2132 can be adjusted by releasing the lock coupling the mounting bracket 2180 to the track 2132, moving the mounting bracket 2180 along the track 2132 to a desired position, and then relocking the lock. The service lines 2152, shown diagrammatically in Fig. 11, extend through the first radial arm 232, the first pivot coupler 236, the second radial arm 234, the second pivot coupler 238, the bore 2208 in the mounting flange 2202, the passageway 2150 (Fig. 14) in the extruded member 2122 for connection to the monitor 282. The service lines 2152 include a low voltage cable coupled to the monitor 282. Appropriate shielding may be provided around the high voltage cable to reduce interference between the low voltage cable and the high voltage cable.

As shown in Fig. 10, the extruded member 2122 is coupled to the pivot coupler 238 by means of a fitting 2196. The fitting 2196 includes a first flange portion 2198 and a second tube portion 2200 that extends generally upwardly from the flange portion 2198. The tube portion 2200 is received in a socket portion 2204 of the pivot coupler 238 for rotation about the vertical axis 276. The fitting 2196 is attached to the extruded member 2122 as explained below. Referring to Fig. 14, the extruded member 2122 has four threaded openings 2253, 2255, 2257, 2259 adjacent to the four corners 2252, 2254, 2256, 2258 of the extruded member 2122 that extend parallel to the longitudinal axis 2110 of the extruded member 2122. Screws (not shown) extend through oversized openings (not shown) in a connector 2199 coupled to fitting 2196 and threadably engage the openings 2253, 2255, 2257, 2259 in the extruded member 2122. In one embodiment, the diameter of the openings 2253, 2255, 2257, 2259 is 0.375 inch (about 0.15 centimeter). The service lines 2152 enter the passageway 2150 in the extruded member 2122 through a central bore 2208 in the flange portion 2198. As shown in Fig. 10, a handle 2220 is coupled to the accessory track 2132 via a mounting bracket 2222 for manipulating the service head 240. Illustratively, the extruded members 2122, 2124 are aluminum extrusions.

The design of the extruded member 2122 is flexible to allow the extruded member 2122 to be interchangeable, reconfigurable and reusable. Depending on the customer's needs, the extruded member 2122 can be cut to various lengths and the accessory tracks 2132, 2134 can have different profiles.

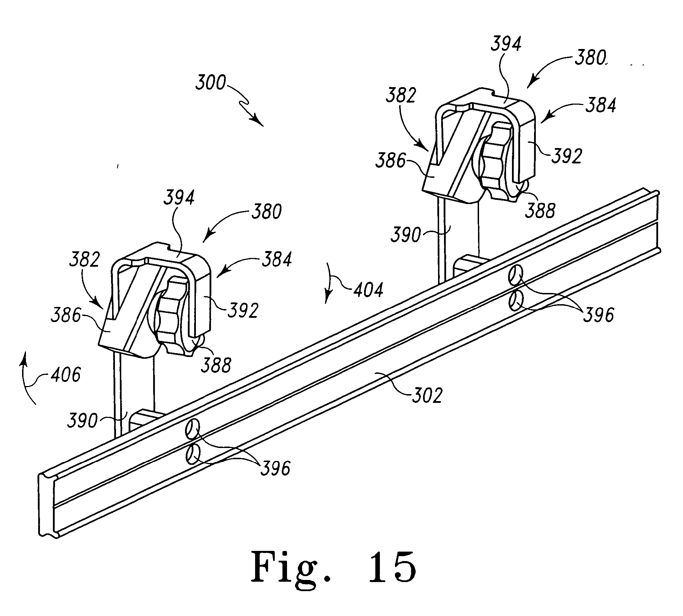

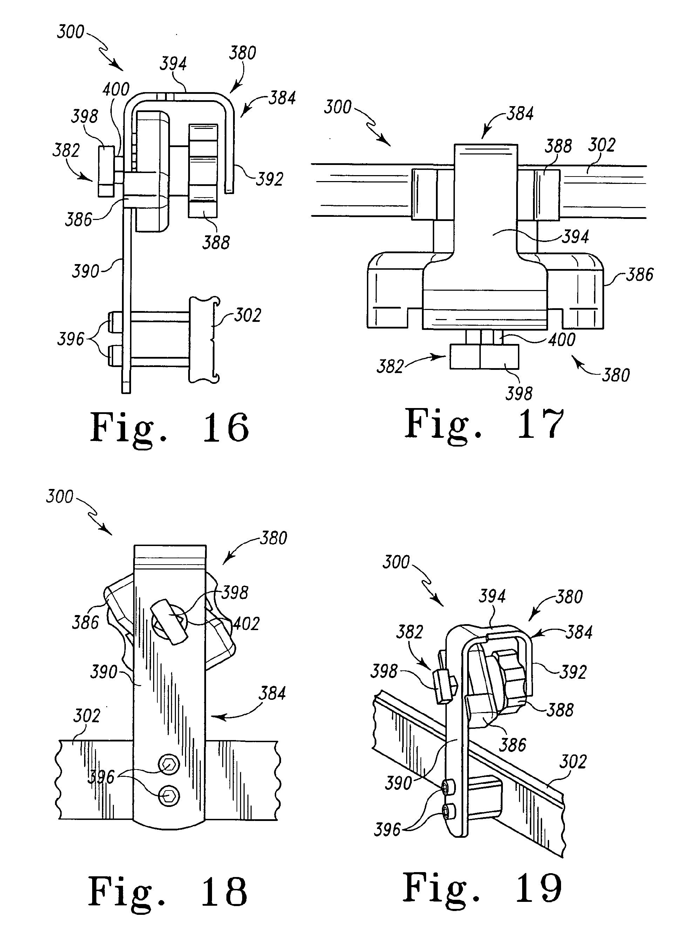

Figs. 15-19 show a Fairfield rail assembly 300 showing a Fairfield rail 302 and two mounting brackets 380 for attaching the Fairfield rail 302 to an accessory track, such as the Hill-Rom profile track 2134 shown in Fig. 14. The Fairfield rail 302 is configured to support various patient care accessories. The mounting bracket 380 comprises a T-bolt 382, a U-shaped bracket 384, an indicator lever 386, and a knob 388. The U-shaped bracket 384 has first and second vertical leg portions 390, 392 having their respective upper ends joined by a horizontal intermediate portion 394. In one illustrative embodiment, the U-shaped bracket 384 is fabricated from sheet metal, and the indicator lever 386 is an injection molded component. The lower end of the vertical leg portion 390 is attached to the Fairfield rail 302 by screws 396. The T-bolt 382 has a head portion 398 and a shaft portion 400 that extends outwardly from the head portion 398. The shaft portion 400 is inserted through an opening 402 (Fig. 18) in the leg portion 390 of the U-shaped bracket 384. The shaft portion 400 has a square cross section portion that is received in a square hole in the indicator lever 386 to key the indicator lever 386 to the T-bolt 382 for rotation therewith. The shaft portion 400 has a threaded portion that is received in a threaded opening in the knob 388. Rotation of the knob 388 in a clockwise direction 404 pulls the head portion 398 of the T-bolt 382 toward the bracket 384. Rotation of the knob 388 in a counterclockwise direction 406 pushes the head portion 398 of the T-bolt 382 away from the bracket 384.

In operation, the head portion 398 of the T-bolt 382 is rotated using the indicator lever 386 to align the longitudinal dimension of the head portion 398 with the longitudinal dimension of the associated accessory track. The head portion 398 of the T-bolt 382 is then inserted into the accessory track The head portion 398 of the T-bolt 382 is then rotated using the indicator lever 386 so that the longitudinal dimension of the head portion 398 is at an angle relative to the longitudinal dimension of the associated accessory track. The knob 388 is rotated in the clockwise direction 404 to attach the mounting bracket 380 to the accessory track. To release the mounting bracket 380, the above procedure is reversed.

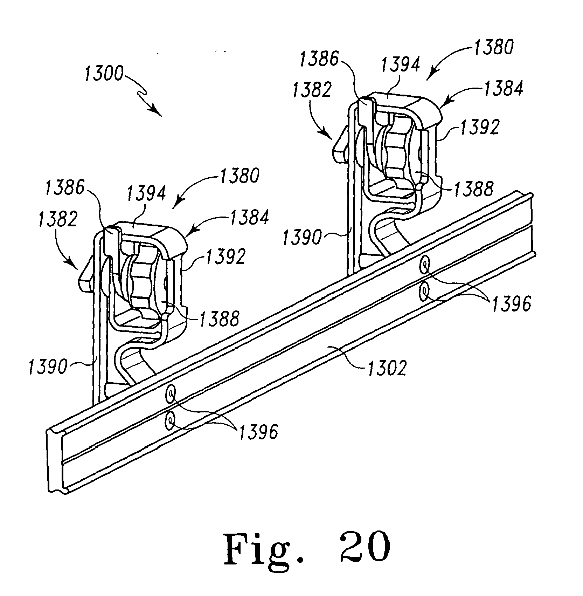

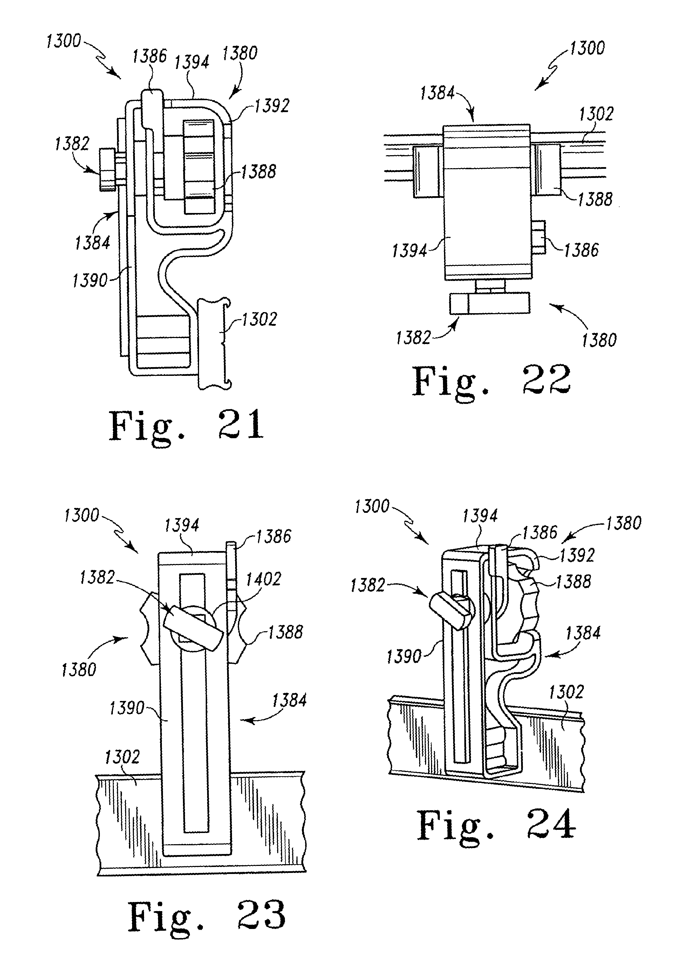

Figs. 20-24 show a second embodiment of a Fairfield rail assembly. Like elements of the two embodiments have generally similar reference numbers. Thus, in the second embodiment, numeral 1300 designates a Fairfield rail assembly, numeral 1302 designates a Fairfield rail, numeral 1380 designates mounting brackets, numeral 1382 designates a T-bolt, numeral 1384 designates a bracket, numeral 1386 designates an indicator lever, and numeral 1388 designates a knob. The operation of the two embodiments is similar. In embodiment illustrated in Figs. 20-24, the U-shaped bracket 1384 and the indicator lever 1386 are injection molded components.

Although the invention has been described in detail with reference to certain illustrative embodiments, it will be appreciated that variations and modifications may be made.

## Claims

1. A service head (40) for use in a healthcare facility, the service head (40) comprising:
a housing (100) having at least one member-receiving cavity (112) opening through an outer surface thereof (102),
at least one extruded member (122) received in the at least one member-receiving cavity (112), the at least one extruded member (122) having an accessory support track (132) that is accessible through an opening (142) in the outer surface (102),
the accessory support track (132) being configured to support at least one patient care accessory (80), and
the housing (100) having an internal passageway (150) for routing at least one service line (152) therethrough to couple to the at least one patient care accessory (80).

2. The service head (40) of claim 1, wherein an outer surface of the at least one extruded member (122) is substantially coplanar with the outer surface of the housing (100).

3. The service head (40) of either claim 1 or claim 2, wherein the housing (100) is a machined aluminum sand casting.

4. The service head (40) of either claim 1 or claim 2, wherein the at least one extruded member (122) is an aluminum extrusion.

5. The service head (40) of any preceding claim, wherein the housing (100) has a first generally vertical portion (190) having the at least one member-receiving cavity (112) and a second portion (192) extending outwardly from an upper end (194) of the first generally vertical portion (190), and the second portion (192) is coupled to a radial arm (34) for pivoting movement therewith.

6. The service head (40) of claim 5, wherein the second portion (192) has a plurality of service connectors (86, 88).

7. The service head (40) of claim 6, wherein the service connectors (86, 88) comprise any one or more of electrical outlets, emergency power outlets, and low voltage outlets.

8. The service head (40) of any one of claims 5 to 7, comprising a mounting flange (202) coupled to the second portion (192) of the housing (100), wherein the mounting flange (202) is coupled to a 2-axes pivot coupler (38) supported by the radial arm (34).

9. The service head (40) of any preceding claim, wherein the at least one member-receiving cavity (112) comprises a first member-receiving cavity (112) opening through a first outer surface (102) of the housing (100) and a second member-receiving cavity (114) opening through a second outer surface (104) of the housing (100), wherein the at least one extruded member (122) comprises a first extruded member (122) received in the first member-receiving cavity (112) and a second extruded member (124) received in the second member-receiving cavity (114), the first extruded member (122) has a first accessory support track (132) that is accessible through a first opening (142) in the first outer surface (102), and the second extruded member (124) has a second accessory support track (134) that is accessible through a second opening (144) in the second outer surface (104).

10. The service head (40) of claim 9, wherein the housing (100) has a front wall (102), a back wall (104), and side walls (106, 108), and the first and second member-receiving cavities (112, 114) open through the front and back walls (102, 104) of the housing (100), respectively.

11. The service head (40) of claim 10, wherein the housing (100) has at least one cross member (126) extending between the side walls (106, 108) of the housing (100), and the first and second extruded members (122, 124) are fastened to the at least one cross member (126) on the opposite sides thereof.

12. A service head (240) for use in a healthcare facility, the service head (240) comprising:
a single-piece extruded member (2122) having a plurality of faces (2242, 2244, 2246, 2248) and a plurality of corners (2252, 2254, 2256, 2258), the plurality of faces (2242, 2244, 2246, 2248) and the plurality of corners (2252, 2254, 2256, 2258) forming a continuous outer surface circumscribing an internal passageway (2150), at least one corner (2252) having an accessory support track (2134) that is accessible through an opening (2144) in the at least one corner (2252),
the accessory track (2134) being configured to support at least one patient care accessory (280), and
the passageway (2150) being configured to allow routing of at least one service line (2152) therethrough to couple to the at least one patient care accessory (280).

13. The service head (40) of claim 12, wherein at least one face (2242) and at least one corner (2252) each has an accessory track (2132, 2134), and the accessory track (2132) in the at least one face (2242) and the accessory track (2134) in the at least one corner (2252) define planes which are generally at 45° angle.

14. The service head (40) of claim 12, wherein the extruded member has four faces (2242, 2244, 2246, 2248) and four corners (2252, 2254, 2256, 2258), and at least one face (2242) has an accessory track (2132).

15. The service head (40) of claim 14, wherein the extruded member (2122) has a generally square cross section, and the passageway (2150) has a generally square cross section.

16. The service head (40) of any one of claims 12 to 15, wherein the extruded member (2122) has at least one opening (2253) adjacent to one of the corners (2252) of the extruded member that extends along a length dimension (2110) of the extruded member (2122) for securing a coupler (2196) to the extruded member (2122).

17. The service head (40) of any one of claims 12 to 16, wherein the accessory track (2134) has a T-shaped cross section comprising a first generally rectangular base portion having a first width and a second generally rectangular head portion having a second width smaller than the first width.

18. A mounting bracket (380) for attaching patient care equipment to an accessory track, the mounting bracket (380) comprising:
a T-bolt 382 having a head portion (398) and a shaft portion (400), the head portion (398) being configured to be coupled to the accessory track, and
an indicator lever (386) mounted on the shaft portion (400) for rotation therewith to indicate the orientation of the T-bolt (382).

19. The mounting bracket (380) of claim 18, further comprising a bracket (384) and a knob (388), wherein the T-bolt (382) is coupled to the bracket (384) for pivoting movement, and the knob (388) threadably engages the shaft portion (400) of the T-bolt (382).

20. The mounting bracket (380) of claim 19, wherein the bracket (384) and the indicator lever (3856) are injection molded.
